# EUROPÄISCHE PATENTSCHRIFT

(11) **EP 0 389 543 B1**
(45) Veröffentlichungstag und Bekanntmachung des Hinweises auf die Patenterteilung: **26.05.1993**
(21) Anmeldenummer: 89900110.1
(22) Anmeldetag: 02.12.1988
(51) Int. Cl.: A61K 49/00, A61B 8/08

(54) **ULTRASCHALL- ODER STOSSWELLENARBEITSVERFAHREN UND MITTEL ZU DESSEN DURCHFÜHRUNG**
ULTRASONIC OR SHOCK WAVE OPERATING PROCESS AND AGENT FOR IMPLEMENTING SAID PROCESS
PROCEDE ET AGENT D'OPERATION PAR ONDES ULTRASONIQUES OU PAR ONDES DE CHOC

(30) Priorität: 02.12.1987 DE 3741201
(43) Veröffentlichungstag der Anmeldung: 03.10.1990
(73) Patentinhaber: SCHERING AKTIENGESELLSCHAFT, 13342 Berlin (DE)
(72) Erfinder: SCHLIEF, Reinhard, D-1000 Berlin 37 (DE)
(86) Internationale Anmeldenummer: DE8800750
(87) Internationale Veröffentlichungsnummer: WO8905159

(56) Entgegenhaltungen:
- EP-A- 0 077 752
- EP-A- 0 122 624
- EP-A- 0 123 235
- EP-A- 0 131 540
- DE-A- 3 637 926
- US-A- 4 276 885

## Beschreibung

Die Erfindung betrifft den im Patentanspruch 1 gekennzeichneten Gegenstand.

In der EP-A-0 284 802 wird eine Zerstörung von biologischen Zellen insbesondere Tumoren beschrieben, bei der die Zellen mit Metallpartikeln markiert und einer Stoßwellen- und/oder Ultraschallapplikation ausgesetzt werden. Diese Mikropartikeln werden durch die Ultraschall- oder Stoßwellenenergie derart beaufschlagt, daß sie schrot- oder schrapnellartig zur Zerstörung des Gewebes beitragen.

Der Erfindung liegt die Aufgabe zugrunde, für die Therapie von Tumorgewebe und/oder Tumorzellen oder andersartiger pathologischer Gewebezellen mit Stoßwellen oder Ultraschallwellen Mittel zur Verfügung zu stellen, die einen synergistischen Effekt ausüben.

Erfindungsgemäß wird diese Aufgabe durch die technische Lehre des Patentanspruchs 1 in Verbindung mit den angegebenen Mitteln gelöst.

Dem Medium, in dem die erfindungsgemäße Wirkung erzielt werden soll, werden die Mikrobläschen erzeugenden oder enthaltenden Mittel appliziert, wodurch die Wirkung eingestrahlter Stoßwellen oder Ultraschallfelder synergistisch verbessert wird. Gleiche Effekte werden mit geringer eingestrahlter Energie erreicht. Bei gleicher Energie ergeben sich somit größere Effekte.

Überrasehenderweise hat sich gezeigt, daß es inbesondere vorteilhaft ist, zur Zerstörung biologischer Gewebe dem Bereich des zu zerstörenden Gewebes mindestens eines der nachstehend aufgeführten Mittel zu applizieren.

Überraschend wurde gefunden, daß die aufgeführten Mittel ausgezeichnete Ergebnisse bei der Stoßwellen und Ultraschalltherapie pathologischer Gewebe, insbesondere Tumore ermöglichen.

Bei Ultraschalleinstrahlung ist es besonders vorteilhaft, die Mikrobläschengröße so einzustellen, daß deren Resonanzfrequenz im Frequenzbereich des Ultraschalls liegt.

Bei dem Verfahren werden die Stoßwellen oder das Ultraschallfeld durch fokusierende Erzeuger in das Medium abgestrahlt, in welches die die Mikrobläschen enthaltenden oder erzeugenden Mittel eingebracht werden.

Stoßwellen oder Ultraschallfelder werden in einen vorbestimmten Bereich mit Vorteil von Erzeugern aus unterschiedlichen Richtungen eingestrahlt. Es ist ferner vorgesehen, den oder die Erzeuger der Stoßwellen oder ultraschallfelder um den Fokus herum zu verschwenken.

In vorteilhafter Weise können
1. eine Supension mit Mikrobläschen, bestehend aus Mikropartikeln aus einer grenzflächenaktiven Substanz in einem flüssigen Träger oder
2. eine Suspension mit Mikrobläschen, bestehend aus Mikropartikeln aus einem nicht grenzflächenaktiven Feststoff in einem flüssigen Träger oder
3. eine Suspension mit Mikrobläschen, bestehend aus Mikropartikeln aus einer Mischung aus mindestens einer grenzflächenaktiven Substanz mit einem nicht grenzflächenaktiven Feststoff in einem flüssigen Träger
für das Stoßwellen- oder Ultraschallverfahren verwendet werden.

Einige dieser erfindungsgemäß zu verwendenden Mittel werden in den EP-OS 122 624 und 123 235 beschrieben.

Mit Vorteil kann das zu verwendende Mittel Mikropartikel enthalten, die als grenzflächenaktive Substanz Lecithine, Polyoxyethylenfettsäureester, Glycerinpolyethylenglykolricinoleat, Polyoxyethylenpolyoxypropylen-Polymere, Saccharoseester, Xyloglyceride, gesättigte oder ungesättigte (C4-C20)-Fettalkohole, gesättigte oder ungesättigte (C4-C20)-Fettsäuren oder deren Salze, Mono-, Di- und Triglyceride, Fettsäureester als Mikropartikel enthalten. Ferner ist es möglich, daß das Mittel Mikropartikel enthält, die als grenzflächenaktiven Stoff Sojaölsaccharoseglycerid oder Polyethylenglykolsorbitanmonostearat enthalten.

Mit besonderem Vorteil können als Mikropartikel Magnesiumstearat, Ascorbylpalmitat, Saccharosemonopalmitat, Saccharosemonostearat, Saccharosedistearat oder Butylstearat als grenzflächenaktive Substanzen im Mittel enthalten sein.

Die Suspension mit Mikrobläschen enthält die grenzflächenaktive Substanz in einer Konzentration von 0,001 bis 5 Gewichtsprozenten, vorzugsweise von 0,04 bis 1 Gewichtsprozent.

Als nicht grenzflächenaktive Feststoffe kann das Mittel mit Vorzug Cyclodextrine, Monosaccharide, Disaccharide, Trisaccharide, Polyole oder anorganische oder organische Salze in einer Konzentration von 2 bis 50 Gewichtsprozenten, vorzugsweise von 9 bis 40 Gewichtsprozenten enthalten. Das Mittel kann ferner Mikropartikel enthalten, die als nicht grenzflächenaktiven Feststoff Dextrose, Maltose, Galactose, Lactose oder α-Cyclodextrin in einer Konzentration von 2 bis 50 Gewichtsprozenten, vorzugsweise 9 bis 40 Gewichtsprozenten, enthalten.

Als geeignete anorganische oder organische Salze sind Natriumchlorid, Natriumcitrat, Natriumacetat oder Natriumtartrat zu nennen.

Mit Vorteil kann das zu verwendende Mittel als flüssigen Träger Wasser, Elektrolytlösung, wäßrige Lösung von ein- oder mehrwertigen Alkoholen oder Polyetheralkoholen oder wäßrige Lösung eines Mono- oder Disaccharids oder Ringer-Lösung oder Tyrode-Lösung oder eine wäßrige Lösung von Maltose, Dextrose, Lactose oder Galactose enthalten. Mit besonderem Vorteil kann der flüssige Träger Glycerin, Polyethylenglykol oder Propylenglykolmethylether enthalten.

Als flüssiger Träger kann aber auch eine Kochsalzlösung in dem Mittel enthalten sein.

Überraschenderweise wurde weiter gefunden, daß ein erfindungsgemäß zu verwendendes Mittel, welches Mikropartikel aus Maltose, Dextrose, Lactose oder Galactose in einem flüssigen Träger der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Lactose oder Galactose sein kann, enthält, ohne Zusatz von viskositätserhöhenden Stoffen wie beispielsweise Propylenglykol eine gute Wirkungsverstärkung bei den Stoßwellen- und Ultraschallverfahren ermöglicht.

Derartige, erfindungsgemäß zu verwendende Mittel werden in der EP-OS 131 540 beschrieben.

Dabei kann das erfindungsgemäß zu benützende Mittel Mikropartikel aus Lactose in bis zu 25 %iger (Gewichtsprozent) wäßriger Lactose-Lösung enthalten. Insbesondere können die erfindungsgemäß zu verwendenden Mittel auch Mikropartikel aus Galactose in bis zu 20 %iger wäßriger Galactose-Lösung oder Mikropartikel aus Galactose in Wasser enthalten.

Ferner ist vorgesehen, daß das Mittel Mikropartikel aus einem Gemisch von Butylstearat und Galactose in Wasser oder aus einem Gemisch von Sojaölsaccharoseglycerid und Galactose in Wasser oder von Polyethylenglykolsorbitanmonostearat und Galactose in physiologischer Kochsalzlösung oder von Ölsäure und Galactose in physiologischer Kochsalzlösung enthält.

Es liegt ferner im Rahmen der Erfindung, daß ein erfindungsgemäß zu verwendendes Mittel eine flüssige Lösung mit Mikrobläschen ist, bestehend aus einem Gemisch von 0,01 bis 10 Gewichtsprozenten eines Tensides oder Tensidgemisches und einer wäßrigen oder mit Wasser mischbaren Trägerflüssigkeit und dem Gemisch von 0,5 bis 50 Gewichtsprozenten einer viskositätserhöhenden Substanz oder eines Substanzgemisches und einer wäßrigen oder mit Wasser mischbaren Trägerflüssigkeit, wobei beide Gemische getrennt oder vereinigt vorliegen.

Mit besonderem Vorteil kann ein Mittel für das Stoßwellen- und Ultraschallverfahren benutzt werden, bestehend aus einem Gemisch von 0,01 bis 10 Gewichtsprozenten eines Tensides oder Tensidgemisches und einer wäßrigen oder mit Wasser mischbaren Trägerflüssigkeit, die 0,05 bis 5 Gewichtsprozente eines physiologisch verträglich carbonsauren Salzes enthält und einem Gemisch von 0,5 bis 50 Gewichtsprozenten einer viskositätserhöhenden Substanz oder eines Substanzgemisches und einer wäßrigen oder mit Wasser mischbaren Trägerflüssigkeit, die dem carbonsauren Salz äquivalente Menge physiologisch verträglicher Säure enthält.

Als carbonsaures Salz findet besonders Natriumhydrogencarbonat und Kaliumhydrogencarbonat Anwendung. Als Säuren sind besonders Milchsäure, Citronensäure und Brenztraubensäure zu nennen.

Derartig erfindungsgemäß zu verwendende Mittel werden in der EP-PS 0077 752 beschrieben.

Als Tenside sind sowohl ionogene als auch nichtionogene Tenside, die gleichzeitig auch viskositätserhöhend wirken können, geeignet. Als nichtionogene Tenside seien genannt: Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerinpolyethylenglykoloxystearat, Glycerinpolyethylenglykolrhizinoleat, ethoxylierte Sojasterine, ethoxylierte Rizinusöle und deren hydrierte Derivate, Cholesterol, Polyoxyethylenpolyoxypropylen-Polymere, wobei Polyoxyethylenfettsäurestearate und Polyoxyethylenpolyoxypropylen-Polymere mit dem Molgewicht 6800-8975, 13300 und 16250 bevorzugt sind.

Als ionogene Tenside kommen in Frage: Quarternäre Ammoniumbasen, Natriumlaurylsulfat, Natriumdioctylsulfosuccinat.

Als viskositätserhöhende Substanzen kommen in Frage: Mono- oder Polysaccharide wie Glucose, Lävulose, Galactose, Lactose, Sorbit, Mannit, Xylit, Saccharose oder Dextrane, Cyclodextrine, Hydroxyethylstärke und Polyole. Als Polyole werden Glycerin, Polyglykole, Inulin und 1,2-Propandiol verwendet. Zur Viskositätserhöhung können weiterhin benutzt werden Proteine, proteinähnliche Stoffe, Aminosäuren oder Blutersatzstoffe wie beispielsweise Plasmaproteine, Gelatine, Oxypolygelatine und Gelatinederivate oder deren Gemische.

Die Konzentration dieser Stoffe in der Lösung kann 0,5 bis 50 Gewichtsprozente betragen, wobei die Höchstkonzentration auch vom gelösten Stoff abhängt. So können beispielsweise Glucose oder Lactose mit einer Konzentration von 0,5 bis 50 Gewichtsprozenten verwendet werden, wogegen Gelatine eine bevorzugte Konzentration von 0,5 bis 2 Gewichtsprozenten hat. Die Oxypolygelatine wird bevorzugt mit einer Konzentration von 0,5 bis 10 Gewichtsprozenten eingesetzt.

Man kann auch Tenside verwenden, die gleichzeitig viskositätserhöhend wirken wie beispielsweise Polyoxyethylenpolyoxpropylen-Polymere mit einem Molekulargewicht von 4750 bis 16250.

In diesem Fall beträgt die Konzentration der Tenside mit viskositätserhöhender Wirkung 1 bis 20 Gewichtsprozenten, vorzugsweise 3 bis 10 Gewichtsprozenten. Das Tensid oder Tensidgemisch wird vorzugsweise in Gegenwart des viskositätserhöhenden Stoffes oder Stoffgemische in einer Trägerflüssigkeit gelöst.

Als Trägerflüssigkeit können Wasser oder wäßrige Lösungen, mit Wasser mischbare ein- oder mehrwertige Alkohole, Ringerlösung, Tyrodelösung oder die wäßrigen Lösungen von Natriumchlorid, Calciumchlorid, Natriumhydrogencarbonat, Natriumcitrat, Natriumacetat oder Natriumtartrat oder Salzlösungen, wie sie üblicherweise als Infusionslösungen verwendet werden, oder deren Gemische verwendet werden.

Weitere erfindungsgemäß zu verwendende Mikrobläschen werden in der US-PS 42 76 885 beschrieben. Diese weisen eine koaleszente widerstandsfähige Oberflächenmembran auf, in der eine Anzahl nicht toxischer und nicht antigener organischer Moleküle angeordnet sind, die ein Gas einer ausgewählten Zusammensetzung enthält, wobei der Durchmesser der Mikrobläschen nicht größer als 300 Micron und nicht kleiner als 0,5 Micron ist. Insbesondere weisen die Moleküle der oberflächenmembran einen hydrophilen und einen hydrophoben Abschnitt auf, wobei deren hydrophiler Abschnitt radial von der Mitte eines Mikrobläschen fortgerichtet ist. Mit besonderem Vorteil wird eine Oberflächenmembran für die Mikrobläschen Verwendet, die aus einer gelierbaren Zusammensetzung, insbesondere Gelatine besteht.

Werden Mikrobläschen, die eine koaleszente widerstandsfähige Oberflächenmembran aufweisen, in der eine Anzahl nicht toxischer und nicht antigener organischer Moleküle angeordnet sind, verwendet, so besteht in vorteilhafter Weise die Möglichkeit, zur gezielten Anwendung dieser Mikrobläschen den Durchmesser auf einen Bereich von 300 Micron bis 0,05 Micron einzustellen. Hierdurch besteht die Möglichkeit, gezielt diese Mikrobläschen bestimmten Körperregionen zu applizieren. Durch den Durchmesser der Bläschen wird festgelegt, in welche Gewebeteile diese Bläschen eindringen können und in welche nicht. Von besonderem Vorteil ist die Verwendung von Mikrobläschen, deren Oberflächenmembran aus einer gelierbaren Zusammensetzung, insbesondere Gelatine besteht.

### Ausführungsbeispiele:

### Beispiel 1:

In einem Wasserbad, welches mit einem Funkenentladungsstoßwellenerzeuger versehen ist, werden in einem Probengefäß in Gelatine eingebettete Leukämiezellen eingebracht. Das Probengefäß wird dann in den zweiten Brennpunkt des Elipsoids gebracht und den Stoßwellen (250 Pulse) ausgesetzt.

### Beispiel 2:

In analoger Weise werden eine gleiche Anzahl (wie in Beispiel 1 genannt) von in Gelatine eingebetteten Leukämiezellen und eine Suspension aus Galactose-Mikropartikeln und Mikrobläschen in einer 20%igen wäßrigen calactoselösung in das Probengefäß eingebracht und ebenfalls 250 Pulsen von Stoßwellen ausgesetzt.

Anschließend werden mittels eines Zellzählapparates (Fluoreszensverfahren) die nach der Stoßwellenbehandlung noch vorhandenen intakten Zellen der Beispiele 1 und 2 gezählt. Dabei wurde festgestellt, daß 0% der ursprünglich vorhandenen Zellen des Beispiels 1 und 15% der Zellen des Beispiels 2 zerstört wurden.

## Patentansprüche

1. Verwendung einer Mikrobläschen enthaltenden Suspension, bestehend aus gasbeladenen Mikropartikeln aus mindestens einer grenzflächenaktiven Substanz und/oder einem nicht grenzflächenalrtiven Feststoff in einem physiologisch verträglichen flüssigen Träger, zur Herstellung eines Präparates für die Stoßwellen- und Ultraschalltherapie pathologischen Gewebes.

2. Verwendung eines Mittels gemäß Anspruch 1, dadurch gekennzeichnet, daß das pathologische Gewebe Tumorgewebe ist.

3. Verwendung eines Mittels nach Anspruch 1 oder 2, enthaltend als grenzflächenaktive Substanz Lecithine, Polyoxyethylenfettsäureester, Polyoxyethylenglykolrizinoleat, Polyoxyethylenpolyoxypropylen-Polymere, Saccharoseester, Xyloglyceride, gesättigte oder ungesättigte (C₄-C₂₀)-Fettalkohole, gesättigte oder ungesättigte (C₄-C₂₀)-Fettsäuren oder deren Salze, Mono-, Di- und Triglyceride, Fettsäureester, Sojaölsaccharoseglycerid oder Polyethylenglykolsorbitanmonostearat als Mikropartikel in einer Konzentration von 0,001 bis 10 Gewichtsprozenten, vorzugsweise 0,04 bis 1 Gewichtsprozent.

4. Verwendung eines Mittels nach einem der Ansprüche 1, 2 oder 3, enthaltend als grenzflächenaktive Substanz Magnesiumstearat, Ascorbylpalmitat, Saccharosemonopalmitat, Saccharosemonostearat, Saccharosedistearat oder Butylstearat in einer Konzentration von 0,001 bis 10 Gewichtsprozenten, vorzugsweise 0,04 bis 1 Gewichtsprozent.

5. Verwendung eines Mittels nach einem der Ansprüche 1 bis 4, enthaltend als nicht grenzflächenaktiven Feststoff Cyclodextrine, Monosaccharide, Disaccharide, Trisaccharide, Polyole oder anorganische oder organische Salze in einer Konzentration von 2 bis 50 Gewichtsprozenten, vorzugsweise von 9 bis 40 Gewichtsprozenten.

6. Verwendung eines Mittels nach einem der Ansprüche 1 bis 5, enthaltend als nicht grenzflächenaktiven Feststoff Galactose, Dextrose, Maltose, Lactose oder α-Cyclodextrin in einer Konzentration von 2 bis 50 Gewichtsprozenten, vorzugsweise von 9 bis 40 Gewichtsprozenten.

7. Verwendung eines Mittels nach einem der Ansprüche 1 bis 6, enthaltend als flüssigen Träger Wasser, physiologische Elektrolytlösung, wäßrige Lösung von ein- oder mehrwertigen Alkoholen oder Polyetheralkoholen oder wäßrige Lösungen eines Mono- oder Disaccharides.

8. Verwendung eines Mittels nach einem der Ansprüche 1 bis 7, enthaltend als flüssigen Träger Ringer-Lösung oder Tyrode-Lösung oder eine wäßrige Lösung von Maltose, Dextrose, Lactose, Galactose, Glycerin, Polyethylenglykol oder Propylenglykolmethylether.

9. Verwendung eines Mittels nach einem der Ansprüche 1 bis 8, enthaltend Mikropartikel bestehend aus einem Gemisch von Butylstearat und Galactose in Wasser.

10. Verwendung eines Mittels nach einem der Ansprüche 1 bis 8, enthaltend Mikropartikel aus Galactose in Wasser.

11. Verwendung eines Mittels nach einem der Ansprüche 1 bis 8, enthaltend Mikropartikel aus Galactose, die in einer bis 20 %igen (Gewichtsprozent) wäßrigen Galactose-Lösung suspendiert sind.

12. Verwendung eines Mittels nach einem der Ansprüche 1 bis 8 enthaltnd Mikropartikel bestehend aus einem Gemsich aus Polyethylenglykolsorbitanmonostearat und Galactose, die in physiologischer Kochsalzlösung suspendiert sind.

13. Verwendung eines Mittels nach einem der Ansprüche 1 bis 8, enthaltend Mikropartikel bestehend aus einem Gemisch aus Ölsäure und Galactose, das in physiologischer Kochsalzlösung suspendiert ist.

14. Verwendung eines Mittels nach einem der Ansprüche 1 bis 8, enthaltend Mikropartikel bestehend aus Lactose die in bis zu 25 %igen (Gewichtsprozent) wäßrigen Lactose-Lösung suspendiert ist.

15. Verwendung einer Mikrobläschen enthaltenden Suspension, bestehend aus
a) einem Gemisch von 0,01 bis 10 Gewichtsprozenten eines Tensides oder Tensidgemisches mit einer wäßrigen oder mit Wasser mischbaren Trägerflüssigkeit und
b) einem Gemisch von 0,5 bis 50 Gewichtsprozenten einer viskositätserhöhenden Substanz oder eines Substanzgemisches und einer wäßrigen oder mit Wasser mischbaren Trägerflüssigkeit,
wobei die Gemische getrennt oder vereinigt vorliegen, zur Herstellung eines Präparates für die Stoßwellen- und Ultraschalltherapie pathologischen Gewebes.

16. Verwendung eines Mittels gemäß Anspruch 15, dadurch gekennzeichnet, daß das pathologische Gewebe Tumorgewebe ist.

17. Verwendung eines Mittels nach Anspruch 15, bestehend aus einem Gemisch von
a) 0,01 bis 10 Gewichtsprozenten eines Tensides oder Tensidgemisches und einer wäßrigen oder mit Wasser mischbaren physiologisch verträglichen Trägerflüssigkeit, die 0,05 bis 5 Gewichtsprozente eines physiologisch verträglichen, carbonsauren Salzes enthält und
b) einem Gemisch von 0,5 bis 50 Gewichtsprozenten einer viskositätserhöhenden Substanz oder eines Substanzgemisches und einer wäßrigen oder mit Wasser mischbaren physiologisch verträglichen Trägerflüssigkeit, die eine dem carbonsauren Salz äquivalente Menge physiologisch verträglicher Säure enthält.

18. Verwendung eines Mittels nach einem der Ansprüche 15 bis 17, enthaltend ein nichtionogenes Tensid, vorzugsweise ein Polyoxyethylenpolyoxypropylen-Polymeres, das gleichzeitig viskositätserhöhend wirkt.

19. Verwendung eines Mittels nach Anspruch 18, enthaltend ein Tensid, das aus einem Polyoxyethylenfettsäureester oder aus einem Polyoxyethylenpolyoxypropylen-Polymeren mit einem Molekulargewicht von 6800 bis 8975 oder 13300 oder 16250 besteht.

20. Verwendung eines Mittels nach einem der Ansprüche 15, 16 oder 17, enthaltend ein Tensid, das aus Polyoxyethylenstearaten oder aus Natriumlaurylsulfat oder Natriumdioctylsulfosuccinat besteht.

21. Verwendung eines Mittels nach Anspruch 15, enthaltend als Trägerflüssigkeit Wasser oder mit Wasser mischbare ein- oder mehrwertige Alkohole, physiologische Elektrolytlösung oder eine Infusionslösung oder deren Gemische.

22. Verwendung einer Mikrobläschen enthaltenden Suspension, bestehend aus gasenthaltenden Mikropartikeln deren Hülle aus einer koaleszenten stabilen Oberflächenmembran besteht, die aus amphoteren organischen Molekülen, bevorzugt Gelatine, aufgebaut ist, wobei der hydrophile Teil der Moleküle radial von der Mitte des Gaskerns fortgerichtet ist und der Durchmesser des Partikels im Bereich von 0,5 bis 300 µm liegt, zur Herstellung eines Präparates für die Stoßwellen und Ultraschalltherapie pathologischen Gewebes.

23. Verwendung eines Mittels gemäß Anspruch 22, dadurch gekennzeichnet, daß das pathologische Gewebe Tumorgewebe ist.

## Claims

1. The use of a suspension containing microbubbles, consisting of gas-charged microparticles of at least one interface-active substance and/or a non-interface-active solid in a physiologically acceptable liquid carrier, for the preparation of a composition for shock-wave therapy and ultrasound therapy of pathological tissue.

2. The use of a composition according to claim 1, characterised in that the pathological tissue is tumour tissue.

3. The use of a composition according to claim 1 or 2, containing as interface-active substance lecithins, polyoxyethylene fatty acid esters, polyoxyethylene glycol ricinoleate, polyoxyethylenepolyoxypropylene polymers, saccharose esters, xylene glycerides, saturated or unsaturated (C₄-C₂₀)fatty alcohols, saturated or unsaturated (C₄-C₂₀)fatty acids or their salts, mono-, di- and tri-glycerides, fatty acid esters, soybean oil saccharose glyceride or polyethylene glycol sorbitan monostearate as microparticles in a concentration of from 0.001 to 10 % by weight, preferably from 0.04 to 1 % by weight.

4. The use of a composition according to any one of claims 1, 2 and 3, containing as interface-active substance magnesium stearate, ascorbyl palmitate, saccharose monopalmitate, saccharose monostearate, saccharose distearate or butyl stearate in a concentration of from 0.001 to 10 % by weight, preferably from 0.04 to 1 % by weight.

5. The use of a composition according to any one of claims 1 to 4, containing as non-interface-active solid cyclodextrins, monosaccharides, disaccharides, trisaccharides, polyols or inorganic or organic salts in a concentration of from 2 to 50 % by weight, preferably from 9 to 40 % by weight.

6. The use of a composition according to any one of claims 1 to 5, containing as non-interface-active solid galactose, dextrose, maltose, lactose or α-cyclodextrin in a concentration of from 2 to 50 % by weight, preferably from 9 to 40 % by weight.

7. The use of a composition according to any one of claims 1 to 6, containing as liquid carrier water, physiological electrolyte solution, aqueous solution of mono- or poly-hydric alcohols or polyether alcohols or aqueous solutions of a mono- or di-saccharide.

8. The use of a composition according to any one of claims 1 to 7 containing as liquid carrier Ringer solution or Tyrode's solution or an aqueous solution of maltose, dextrose, lactose, galactose, glycerol, polyethylene glycol or propylene glycol methyl ether.

9. The use of a composition according to any one of claims 1 to 8 containing microparticles consisting of a mixture of butyl stearate and galactose in water.

10. The use of a composition according to any one of claims 1 to 8 containing microparticles of galactose in water.

11. The use of a composition according to any one of claims 1 to 8 containing microparticles of galactose that are suspended in an up to 20 % (by weight) aqueous galactose solution.

12. The use of a composition according to any one of claims 1 to 8 containing microparticles consisting of a mixture of polyethylene glycol sorbitan, monostearate and galactose that are suspended in physiological saline.

13. The use of a composition according to any one of claims 1 to 8 containing microparticles consisting of a mixture of oleic acid and galactose that is suspended in physiological saline.

14. The use of a composition according to any one of claims 1 to 8 containing microparticles consisting of lactose that is suspended in an up to 25 % (by weight) aqueous lactose solution.

15. The use of a suspension containing microbubbles, consisting of
a) a mixture of 0.01 to 10 % by weight of a surfactant or surfactant mixture with an aqueous or water-miscible carrier liquid and
b) a mixture of from 0.5 to 50 % by weight of a viscosity-increasing substance or substance mixture and an aqueous or water-miscible carrier liquid,
wherein the mixtures are present separately or combined, for the preparation of a composition for shock-wave therapy and ultrasound therapy of pathological tissue.

16. The use of a composition according to claim 15, characterised in that the pathological tissue is tumour tissue.

17. The use of a composition according to claim 15, consisting of a mixture of
a) from 0.01 to 10 % by weight of a surfactant or surfactant mixture and an aqueous or water-miscible physiologically acceptable carrier liquid that contains from 0.05 to 5 % by weight of a physiologically acceptable carboxylic acid salt and
b) a mixture of from 0.5 to 50 % by weight of a viscosity-increasing substance or substance mixture and an aqueous or water-miscible physiologically acceptable carrier liquid that contains an amount of physiologically acceptable acid that is equivalent to the carboxylic acid salt.

18. The use of a composition according to any one of claims 15 to 17, containing a non-ionic surfactant, preferably a polyoxyethylenepolyoxypropylene polymer, that simultaneously acts as a viscosity-increasing substance.

19. The use of a composition according to claim 18, containing a surfactant that consists of a polyoxyethylene fatty acid ester or of a polyoxyethylenepolyoxypropylene polymer with a molecular weight of from 6800 to 8975 or 13300 or 16250.

20. The use of a composition according to any one of claims 15, 16 and 17, containing a surfactant that consists of polyoxyethylene stearates or of sodium lauryl sulphate or sodium dioctylsulphosuccinate.

21. The use of a composition according to claim 15, containing as carrier liquid water or water-miscible mono- or poly-hydric alcohols, physiological electrolyte solution or an infusion solution or mixtures thereof.

22. The use of a suspension containing microbubbles, consisting of gas-containing microparticles of which the casing consists of a coalescent stable surface membrane constructed from amphoteric organic molecules, preferably gelatin, wherein the hydrophilic moiety of the molecule projects radially from the centre of the gas core and the diameter of the particle is in the range of from 0.5 to 300 µm, for the preparation of a composition for shock-wave and ultrasound therapy of pathological tissue.

23. The use of a composition according to claim 22, characterised in that the pathological tissue is tumour tissue.

## Revendications

1. Emploi d'une suspension contenant des microbulles, constituée de microparticules chargées d'un gaz, d'au moins une substance tensio-active et/ou d'une matière solide non-tensio-active, dans un véhicule liquide physiologiquement compatible, pour en fabriquer une préparation destinée au traitement thérapeutique par ondes de choc et par ultrasons de tissus malades.

2. Emploi d'un produit selon la revendication 1, caractérisé en ce que le tissu malade est un tissu tumoral.

3. Emploi d'un produit selon la revendication 1 ou 2 qui comprend comme substance tensio-active une lécithine, un ester de polyoxyéthylène-acide gras, un ricinoléate de polyoxyéthylène-glycol, un polymère d'oxydes d'éthylène et de propylène, un ester de saccharose, un glycéride de xylène, un alcool gras saturé ou insaturé en C4-C20, un acide gras saturé ou insaturé en C₄-C₂₀ ou un sel de cet acide, un mono- di- ou triglycéride, un ester d'acide gras, un glycéride d'huile de soja et de saccharose ou un monostéarate de polyéthylène-glycol-sorbitanne, en microparticules à une concentration de 0,001 à 10% en poids, de préférence de 0,04 à 1%.

4. Emploi d'un produit selon l'une des revendications précédentes qui comprend comme substance tensio-active du stéarate de magnésium, du palmitate d'ascorbyle, du monopalmitate de saccharose, du monostéarate ou du distéarate de saccharose ou du stéarate de buyle à une concentration de 0,001 à 10% en poids, de préférence de 0,04 à 1%.

5. Emploi d'un produit selon l'une des revendications précédentes qui comprend comme matière solide non-tensio-active des cyclodextrines, des monoholosides, diholosides ou triholosides, des polyols ou des sels minéraux ou organiques à une concentration de 2 à 50% en poids, de préférence de 9 à 40%.

6. Emploi d'un produit selon l'une des revendications précédentes qui comprend comme matière solide non-tension-active du galactose, du dextrose, du maltose, du lactose ou de l'α-cyclodextrine à une concentration de 2 à 50% en poids, de préférence de 9 à 40%.

7. Emploi d'un produit selon l'une des revendications précédentes qui comprend comme véhicule liquide de l'eau, une solution d'électrolyte physiologique, une solution aqueuse de mono-alcools ou de poly-alcools ou de polyéthers-alcools ou une solution aqueuse d'un monoholoside ou d'un diholoside.

8. Emploi d'un produit selon l'une des revendications précédentes qui comprend comme véhicule liquide une solution de Ringer ou de Tyrode ou une solution aqueuse de maltose, dextrose, lactose, galactose, glycérol, d'un polyéthylène-glycol ou d'éther méthylique de propylène-glycol.

9. Emploi d'un produit selon l'une des revendications précédentes qui comprend des microparticules d'un mélange de stéarate de butyle et de galactose dans de l'eau.

10. Emploi d'un produit selon l'une des revendications 1 à 8 qui comprend des microparticules de galactose dans de l'eau.

11. Emploi d'un produit selon l'une des revendications 1 à 8 qui comprend des microparticules de galactose en suspension dans une solution aqueuse de galactose à une teneur pouvant s'élever jusqu'à 20% en poids.

12. Emploi d'un produit selon l'une des revendications 1 à 8 qui comprend des microparticules d'un mélange d'un monostéarate de polyéthylène-glycol-sorbitanne et de galactose en suspension dans une solution de sérum physiologique.

13. Emploi d'un produit selon l'une des revendications 1 à 8 qui comprend des microparticules d'un mélange d'acide oléique et de galactose en suspension dans du sérum physiologique.

14. Emploi d'un produit selon l'une des revendication 1 à 8 qui comprend des microparticules de lactose en suspension dans une solution aqueuse de lactose à une teneur pouvant s'élever jusqu'à 25% en poids.

15. Emploi d'une suspension de microbulles constituée de :
a) un mélange de 0,01 à 10% en poids d'un ou de plusieurs agents tensio-actifs et d'un liquide véhicule aqueux ou miscible à l'eau, et
b) un mélange de 0,5 à 50% en poids d'une ou plusieurs matières élevant la viscosité et d'un liquide véhicule aqueux ou miscible à l'eau,
mélanges qui sont séparés ou réunis, pour en former une préparation destinée au traitement thérapeutique de tissus malades par ondes de choc et ultrasons.

16. Emploi d'un produit selon la revendication 15, caractérisé en ce que le tissu malade est un tissu tumoral.

17. Emploi d'un produit selon la revendication 15 constitué d'un mélange de :
a) 0,01 à 10% en poids d'un ou de plusieurs agents tensio-actifs et d'un véhicule liquide physiologiquement toléré, aqueux ou miscible à l'eau, contenant de 0,05 à 5% en poids d'un sel d'acide carboxylique physiologiquemen toléré, et
b) un mélange de 0,5 à 50% en poids d'une ou plusieurs matières élevant la viscosité et d'un véhicule liquide physiologiquement toléré, aqueux ou miscible à l'eau, contenant une proportion d'acide physiologiquement toléré équivalente au sel de l'acide carboxylique.

18. Emploi d'un produit selon l'une des revendications 15 à 17 contenant un agent tensio-actif non-ionogène, de préférence un polymère d'oxydes d'éthylène et de propylène, qui en même temps accroît la viscosité.

19. Emploi d'un produit selon la revendication 18 contenant un agent tensio-actif formé d'un ester de polyoxyéthylène-acide gras ou d'un polymère d'oxydes d'éthylène et de propylène d'une masse moléculaire de 6800 à 9875 ou 13300 ou 16250.

20. Emploi d'un produit selon l'une des revendications 15, 16 et 17 contenant un agent tesio-actif formé de stéarates de polyoxyéthylènes ou bien de laurylsulfate de sodium ou du sel sodique de sulfosuccinate de dioctyle.

21. Emploi d'un produit selon la revendication 15 comprenant comme liquide véhicule de l'eau ou des mono-alcools ou poly-alcools miscibles à l'eau, une solution d'électrolyte physiologique ou une solution d'infusion ou encore un mélange de ceux-ci.

22. Emploi d'une suspension de microbulles constituée de microparticules contenant un gaz dont l'enveloppe est formée d'une membrane de surface stable coalescente, de molécules organiques amphotères, de préférence de gélatine, la partie hydrophile de ces molécules étant orientée radialement depuis le milieu du coeur de gaz et le diamètre des particules étant de 0,5 à 300 µm, pour en former une préparation destinée au traitement thérapeutique de tissus malades par ondes de choc et ultrasons.

23. Emploi d'un produit selon la revendication 22, caractérisé en ce que le tissu malade est un tissu tumoral.
